# EUROPEAN PATENT APPLICATION

(11) **EP 4 563 706 A1**
(43) Date of publication of application: **04.06.2025**
(21) Application number: 23846943.1
(22) Date of filing: 24.07.2023
(51) Int. Cl.: C12P 7/625, C12N 9/50

(54) **METHOD FOR PREPARING PURIFIED POLYHYDROXYALKANOATE**

(30) Priority: 25.07.2022 KR 20220091710
(71) Applicant: CJ CheilJedang Corporation, Seoul 04560 (KR)
(72) Inventor: HONG, Chang Young, Seoul 04560 (KR); SEO, Dong June, Seoul 04560 (KR); KIM, Da Eun, Seoul 04560 (KR); LEE, Han-Sol, Seoul 04560 (KR); LEE, Ilgyu, Seoul 04560 (KR); KIM, Jung Min, Seoul 04560 (KR)
(74) Representative: Meissner Bolte Partnerschaft mbB
(86) International application number: PCT/KR2023/010686
(87) International publication number: WO 2024/025284

(57) **Abstract**

A method for preparing purified polyhydroxyalkanoate of the present invention may lower the b* value of a final product more than the conventional value by means of a decolorization step using two types of oxidizing agents sequentially. In addition, in the present invention, by using sodium chlorite (NaClO₂), as a primary oxidizing agent, the effect of improving color is significant, and there is almost no decrease in molecular weight.

## Description

### Technical Field

The present disclosure relates to a process for preparing a purified polyhydroxyalkanoate, and, particularly, to a process for preparing a purified polyhydroxyalkanoate having excellent color through a decolorization process using an oxidizing agent.

### Background Art

Polyhydroxyalkanoates (PHAs) are biodegradable polymers composed of several types of hydroxyl carboxylic acids produced by numerous microorganisms and used as intracellular storage materials. Polyhydroxyalkanoates have physical properties similar to those of conventional petroleum-derived synthetic polymers such as polybutylene adipate terephthalate (PBAT), polybutylene succinate (PBS), polybutylene succinate terephthalate (PBST), and polybutylene succinate adipate (PBSA), exhibit complete biodegradability, and are excellent in biocompatibility.

Specifically, the polyhydroxyalkanoate is a natural thermoplastic polyester polymer that accumulates in microbial cells. Since it is a biodegradable material, it can be composted and ultimately decomposed into carbon dioxide, water, and organic waste without generating toxic waste. In particular, the polyhydroxyalkanoate is biodegradable under any environmental conditions, such as soil and sea, and has environmentally friendly characteristics.

### [Prior Art Document]

(Patent Document 1) Korean Laid-open Patent Publication No. 2001-0009719

### Disclosure of Invention

### Technical Problem

To obtain a polyhydroxyalkanoate (PHA), a fermentation broth obtained from microorganisms is subjected to cell crushing and separation to prepare a crude product. Then, it is necessary to remove impurities through a purification process such as decolorization to lower the color of the final PHA. However, in the case of a PHA having flexibility and tackiness, there is a problem in that the impurities attached to the PHA particles are not removed, and the particle size of the PHA remains at a low level in the purification process, making difficult the subsequent repeated solid-liquid separation and washing, whereby the content of impurities in the final PHA is still high. As described above, the conventional purification methods of PHAs are unable to effectively reduce the content of impurities in some PHAs, resulting in an increase in the b* value of a purified final PHA to 15 or higher, which makes the commercialization thereof difficult. This problem is more severe for some PHAs with low or no crystallinity.

As a result of research conducted by the present inventors, it has been discovered that the color of a final product can be improved by sequentially performing decolorization using two types of oxidizing agents during the purification process of polyhydroxyalkanoates, thereby resolving the problems of the conventional purification methods.

Accordingly, an object of the present disclosure is to provide a process for preparing a purified polyhydroxyalkanoate having excellent color, which comprises a decolorization step using an oxidizing agent.

### Solution to Problem

According to an aspect of the present disclosure, there is provided a process for preparing a purified polyhydroxyalkanoate, which comprises preparing a crude product comprising a polyhydroxyalkanoate (PHA); first decolorizing the crude product to obtain a first product; and second decolorizing the first product to obtain a second product, wherein the first decolorization is carried out with a first oxidizing agent comprising sodium chlorite (NaClO₂).

In an embodiment, the second decolorization may be carried out with a second oxidizing agent comprising hydrogen peroxide (H₂O₂).

In another embodiment, the first decolorization may be carried out at a pH of 2 to 7, and the second decolorization may be carried out at a pH of 8 to 13.

In another embodiment, in the first decolorization, the sodium chlorite may be used in an amount of 0.01 to 1% by weight based on the total weight of the crude product.

In another embodiment, in the second decolorization, the hydrogen peroxide may be used in an amount of 0.01 to 1% by weight based on the total weight of the first product.

In another embodiment, the first product may have a content of lipids of less than 1% by weight based on the weight of the total solids content.

In another embodiment, the process may further comprise deproteinizing the second product using a protease.

In another embodiment, in the deproteinization, the protease may be used in an amount of 0.001 to 0.1% by weight based on the total weight of the second product.

In another embodiment, the deproteinization may be carried out at a pH of 7 to 11.

In another embodiment, the crude product comprising a polyhydroxyalkanoate may be prepared by a method comprising performing a solid-liquid separation of a fermentation broth comprising a polyhydroxyalkanoate (PHA) to obtain biomass; mixing an additive comprising a surfactant with the biomass to obtain a suspension; crushing the biomass in the suspension to obtain a slurry; and performing a solid-liquid separation of the slurry.

According to another aspect of the present disclosure, there is provided a polyhydroxyalkanoate, which is prepared by the above process and has a b* value of less than 15 in the CIELAB color coordinates.

In an embodiment, the crystallization temperature (Tc) of the polyhydroxyalkanoate may not be measured or may be measured to be 60°C to 120°C.

In another embodiment, the repeat unit that constitutes the polyhydroxyalkanoate may comprise at least one selected from the group consisting of 2-hydroxybutyrate (2-HB), 3-hydroxybutyrate (3-HB), 3-hydroxypropionate (3-HP), 3-hydroxyvalerate (3-HV), 3-hydroxyhexanoate (3-HH), 3-hydroxyheptanoate (3-HHep), 3-hydroxyoctanoate (3-HO), 3-hydroxynonanoate (3-HN), 3-hydroxydecanoate (3-HD), 3-hydroxydodecanoate (3-HDd), 4-hydroxybutyrate (4-HB), 4-hydroxyvalerate (4-HV), 5-hydroxyvalerate (5-HV), and 6-hydroxyhexanoate (6-HH).

### Advantageous Effects of Invention

According to the process of the present disclosure, the b* value of a final product is lowered to less than 15 through a decolorization step sequentially using two types of oxidizing agents, whereby it is possible to solve the problem of an increase in color that may occur in the conventional water-based purification process of a polyhydroxyalkanoate (PHA). In addition, the sequential use of two types of oxidizing agents to solve the conventional color problem as described above can reduce the amount of enzyme used, thereby reducing the production cost, which is economical.

In particular, sodium chlorite (NaClO₂) used as a first oxidizing agent in the present disclosure produces a significant effect of color improvement, while it causes almost no change in polymer properties through a decrease in molecular weight, unlike other chlorine-based oxidizing agents (such as NaClO). Accordingly, the purified polyhydroxyalkanoate obtained by the process of the present disclosure has improved color and purity as compared with conventional polyhydroxyalkanoates, while it also has an excellent effect in maintaining molecular weight.

In particular, the effects of the present disclosure described above can be more significantly produced in the purification process of an amorphous PHA. When the process of the present disclosure is applied to the purification process of other crystalline and semi-crystalline PHAs, the effects of improving color and purity and maintaining molecular weight can also be achieved.

### Brief Description of Drawings

Fig. 1 shows the process for preparing a purified polyhydroxyalkanoate according to an embodiment of the present disclosure.
Fig. 2 illustrates an example of a process for preparing a crude product of a polyhydroxyalkanoate.

### Best Mode for Carrying out the Invention

Hereinafter, the present disclosure will be described in more detail with reference to various embodiments.

In this specification, terms referring to the respective components are used to distinguish them from each other and are not intended to limit the scope of the present disclosure. In addition, in the present specification, a singular expression is interpreted to cover a plural number as well unless otherwise specified in the context.

In the present specification, the term "comprising" is intended to specify a particular characteristic, region, step, process, element, and/or component. It does not exclude the presence or addition of any other characteristic, region, step, process, element and/or component, unless specifically stated to the contrary.

In the present specification, the terms first, second, and the like are used to describe various components. But the components should not be limited by the terms. The terms are used for the purpose of distinguishing one element from another.

In the present specification, molecular weight or weight average molecular weight is usually not described with units, but it may be understood that it has a unit of g/mole or Da.

### Process for preparing a purified polyhydroxyalkanoate

The process for preparing a purified polyhydroxyalkanoate according to an embodiment comprises preparing a crude product comprising a polyhydroxyalkanoate (PHA); first decolorizing the crude product to obtain a first product; and second decolorizing the first product to obtain a second product, wherein the first decolorization is carried out with a first oxidizing agent comprising sodium chlorite (NaClO₂).

The process may further comprise deproteinizing the second product using a protease.

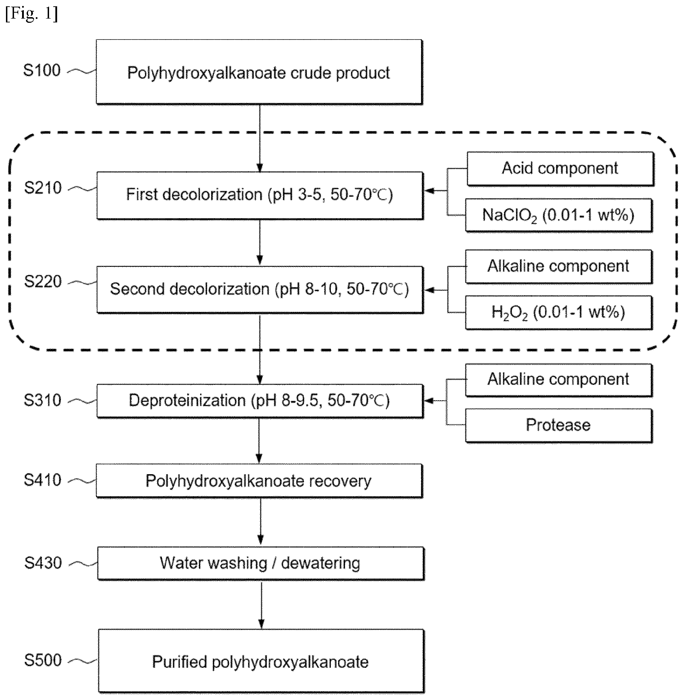

Fig. 1 shows the process for preparing a purified polyhydroxyalkanoate according to an embodiment of the present disclosure.

Referring to Fig. 1, according to an embodiment, a crude product of a polyhydroxyalkanoate is prepared (S100). An acid is added to the crude product to adjust the pH to about 3 to 5, and sodium chlorite (NaClO₂) is added to perform first decolorization at about 50 to 70°C (S210). An alkaline component is added to the product to adjust the pH to about 8 to 10, and hydrogen peroxide (H₂O₂) is added to perform second decolorization at about 50 to 70°C (S220). An alkaline component is added to the decolorized product to adjust the pH to about 8 to 9.5, and deproteinization is carried out using a protease (S310). After the deproteinization, the PHA solid is recovered (410). Water washing and dewatering are carried out (S430) to obtain a purified final polyhydroxyalkanoate (S500).

Meanwhile, the crude product of a polyhydroxyalkanoate used as a starting material in the present disclosure may be prepared from a fermentation broth through processes such as filtration, crushing, and purification.

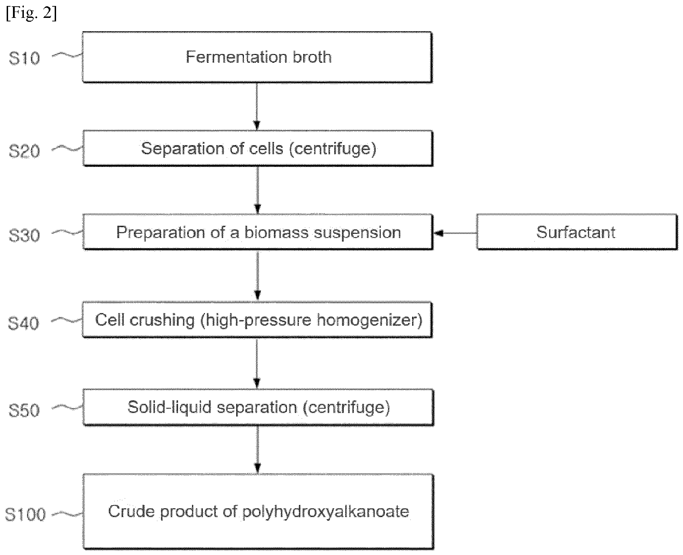

Fig. 2 illustrates an example of a process for preparing a crude product of a polyhydroxyalkanoate.

Referring to Fig. 2, according to an embodiment, a fermentation broth of a polyhydroxyalkanoate is prepared (S10). The cells are separated by centrifugation or the like (S20). A surfactant or the like is added (S30). The cells are crushed by a high-pressure homogenizer (S40). A solid-liquid separation is carried out by centrifugation or the like (S50). A crude product of a polyhydroxyalkanoate is obtained (S100).

Hereinafter, each step of the preparation process according to the present disclosure will be described in detail.

### Polyhydroxyalkanoate

Polyhydroxyalkanoate (PHA) may be classified as crystalline, semi-crystalline, or amorphous polyhydroxyalkanoate, depending on its molecular structure.

According to an embodiment, the polyhydroxyalkanoate may comprise an amorphous polyhydroxyalkanoate. In the case of an amorphous polyhydroxyalkanoate, repeated solid-liquid separation and washing are difficult during the water-based purification process due to its unique flexibility and tackiness, whereby the content of impurities in a purified final PHA is still high, making difficult the commercialize thereof. According to the present disclosure, however, as decolorization using two types of oxidizing agents is sequentially carried out during the purification process of an amorphous PHA, the color of a final product can be significantly improved.

According to another embodiment, the polyhydroxyalkanoate may comprise a semi-crystalline polyhydroxyalkanoate. According to another embodiment, the polyhydroxyalkanoate may comprise a crystalline polyhydroxyalkanoate. According to another embodiment, the polyhydroxyalkanoate may comprise two or more of an amorphous polyhydroxyalkanoate, a semi-crystalline polyhydroxyalkanoate, and a crystalline polyhydroxyalkanoate.

The polyhydroxyalkanoate may comprise at least one repeat unit selected from the group consisting of 2-hydroxybutyrate (2-HB), 3-hydroxybutyrate (3-HB), 3-hydroxypropionate (3-HP), 3-hydroxyvalerate (3-HV), 3-hydroxyhexanoate (3-HH), 3-hydroxyheptanoate (3-HHep), 3-hydroxyoctanoate (3-HO), 3-hydroxynonanoate (3-HN), 3-hydroxydecanoate (3-HD), 3-hydroxydodecanoate (3-HDd), 4-hydroxybutyrate (4-HB), 4-hydroxyvalerate (4-HV), 5-hydroxyvalerate (5-HV), and 6-hydroxyhexanoate (6-HH).

In addition, the polyhydroxyalkanoate may comprise isomers. For example, the polyhydroxyalkanoate may comprise structural isomers, enantiomers, or geometric isomers. Specifically, the polyhydroxyalkanoate may comprise structural isomers.

The polyhydroxyalkanoate may be a homopolymer or a copolymer. According to an embodiment, the polyhydroxyalkanoate may comprise a copolymer, specifically, a copolymer comprising two or more different repeat units with the different repeat units randomly distributed in the polymer chain.

As an example, the polyhydroxyalkanoate may comprise a 4-hydroxybutyrate (4-HB) repeat unit. For example, the content of a 4-hydroxybutyrate (4-HB) repeat unit in the polyhydroxyalkanoate may be 0.1% by weight or more, 1% by weight or more, 5% by weight or more, 10% by weight or more, 20% by weight or more, 30% by weight or more, 40% by weight or more, 50% by weight or more, or 60% by weight or more, and 100% by weight or less, 99% by weight or less, 90% by weight or less, 80% by weight or less, 70% by weight or less, or 60% by weight or less, based on the total weight of the polyhydroxyalkanoate. Specifically, the polyhydroxyalkanoate may comprise a 4-hydroxybutyrate (4-HB) repeat unit in an amount of 25% by weight or more.

Specifically, the polyhydroxyalkanoate may be a copolymer comprising a 4-HB repeat unit. Examples of the repeat units that may be contained in the polyhydroxyalkanoate, together with 4-HB, may include at least one selected from the group consisting of lactic acid, glycolic acid, 2-hydroxybutyrate (2-HB), 3-hydroxybutyrate (3-HB), 3-hydroxypropionate (3-HP), 3-hydroxyvalerate (3-HV), 3-hydroxyhexanoate (3-HH), 3-hydroxyheptanoate (3-HHep), 3-hydroxyoctanoate (3-HO), 3-hydroxynonanoate (3-HN), 3-hydroxydecanoate (3-HD), 3-hydroxydodecanoate (3-HDd), 4-hydroxyvalerate (4-HV), 5-hydroxyvalerate (5-HV), and 6-hydroxyhexanoate (6-HH).

More specifically, the polyhydroxyalkanoate may comprise one or more repeat units selected from the group consisting of 3-HB, 3-HP, 3-HH, 3-HV, 4-HV, 5-HV, and 6-HH, in addition to a 4-HB repeat unit. As an example, the polyhydroxyalkanoate may comprise a copolymer comprising a 4-HB repeat unit and a 3-HB repeat unit. Specifically, it may comprise poly-3-hydroxybutyrate-co-4-hydroxybutyrate (P3HB-co-4HB).

The PHA may comprise one or more types of PHAs. For example, it may comprise a first PHA, a second PHA, or a mixture thereof. For example, the first PHA may be an amorphous PHA, and the second PHA may be a semi-crystalline PHA. The first PHA may comprise a 4-HB repeat unit in an amount of, for example, 15% by weight to 60% by weight, 15% by weight to 55% by weight, 20% by weight to 55% by weight, 25% by weight to 55% by weight, 30% by weight to 55% by weight, 35% by weight to 55% by weight, 20% by weight to 50% by weight, 25% by weight to 50% by weight, 30% by weight to 50% by weight, 35% by weight to 50% by weight, or 20% by weight to 40% by weight. The second PHA may comprise a 4-HB repeat unit in an amount of, for example, 0.1% by weight to 30% by weight, 0.5% by weight to 30% by weight, 1% by weight to 30% by weight, 3% by weight to 30% by weight, 1% by weight to 28% by weight, 1% by weight to 25% by weight, 1% by weight to 24% by weight, 1% by weight to 20% by weight, 1% by weight to 15% by weight, 2% by weight to 25% by weight, 3% by weight to 25% by weight, 3% by weight to 24% by weight, 5% by weight to 24% by weight, 5% by weight to 20% by weight, greater than 5% by weight to less than 20% by weight, 7% by weight to 20% by weight, 10% by weight to 20% by weight, 15% by weight to 25% by weight, or 15% by weight to 24% by weight. In addition, the PHA may be a mixture of the first PHA and the second PHA at a weight ratio of 20:80 to 80:20 or 30:70 to 70:30.

### Crude product of a polyhydroxyalkanoate

In this step, a crude product of a polyhydroxyalkanoate is prepared.

In general, a polyhydroxyalkanoates can be produced by a fermentation process. The crude product of a polyhydroxyalkanoate may also be obtained through fermentation.

The method for producing a polyhydroxyalkanoate (PHA) through fermentation may be carried out by a known method. For example, a polyhydroxyalkanoate can be produced through fermentation using wild-type or transgenic microorganisms cultured on a specific substrate, and it is not particularly limited.

Upon fermentation, the polyhydroxyalkanoate thus produced accumulates within microbial cells; thus, a process of separating and purifying the same is absolutely necessary.

For example, biomass containing a polyhydroxyalkanoate is separated from the fermentation broth, a surfactant is added thereto, and the microbial cells are crushed to separate the polyhydroxyalkanoate only.

As an example, the crude product comprising a polyhydroxyalkanoate may be prepared by a method comprising performing a solid-liquid separation of a fermentation broth comprising a polyhydroxyalkanoate (PHA) to obtain biomass; mixing an additive comprising a surfactant with the biomass to obtain a suspension; crushing the biomass in the suspension to obtain a slurry; and performing a solid-liquid separation of the slurry.

First, a fermentation broth formed by microorganisms is subjected to a solid-liquid separation using a mechanical separation method such as centrifugation and membrane separation or other known methods to separate biomass comprising cells in which a polyhydroxyalkanoate (PHA) has accumulated.

The biomass thus separated may be mixed with a solvent to adjust the solids concentration. The biomass may be mixed with a surfactant or the like. For example, an anionic surfactant or a nonionic surfactant may be used as the surfactant. Specifically, sodium laureth sulfate, sodium lauryl sulfate, dodecyl sodium sulfate, and dodecyl sodium benzene sulfonate may be used. As a result, a suspension comprising biomass, surfactant, or the like may be obtained.

Thereafter, the biomass in the suspension is crushed, and the crushing may be carried out by mechanical crushing (physical crushing) or chemical crushing (nonmechanical crushing). For example, the crushing may be carried out by ultrasonic crushing, high-pressure crushing, or milling crushing. The ultrasonic crushing may be carried out, for example, for 10 minutes to 60 minutes at an energy level of 20 Hz or higher. In addition, the high-pressure crushing may be carried out, for example, for 1 minute to 60 minutes at a pressure of 10 bar or more. In addition, the milling crushing may be carried out for 1 minute to 60 minutes by a colloid mill, a bead mill, or a ball mill.

As a result of crushing, a slurry comprising fine particles of a polyhydroxyalkanoate and cell-derived impurities such as cell walls may be obtained. The slurry is subjected to a solid-liquid separation using a mechanical separation method such as centrifugation and membrane separation or other known methods to obtain a crude product of a polyhydroxyalkanoate.

Since the crude product of a polyhydroxyalkanoate comprises trace amounts of cell-derived impurities (proteins, lipids, and the like), it requires further purification.

For example, the content of proteins in the crude product may be 3.5% by weight or less, 3% by weight or less, 2.7% by weight or less, or 2.5% by weight or less, and may be 0.1% by weight or more, 1% by weight or more, or 1.5% by weight or more, based on the total solids weight in the crude product.

In addition, the content of lipids in the crude product may be 1.5% by weight or less, 1.3% by weight or less, 1% by weight or less, or 0.7% by weight or less, and may be 0.1% by weight or more, 0.2% by weight or more, or 0.3% by weight or more, based on the total solids weight in the crude product.

In addition, the solids content in the crude product may be 1% by weight or more, 3% by weight or more, 5% by weight or more, 7% by weight or more, or 10% by weight or more, and may be 80% by weight or less, 50% by weight or less, 30% by weight or less, 20% by weight or less, or 15% by weight or less, based on the total weight of the crude product.

### First decolorization

In this step, the crude product comprising a polyhydroxyalkanoate is subjected to first decolorization to obtain a first product.

The first decolorization is carried out by a first oxidizing agent comprising sodium chlorite (NaClO₂). When decolorization is carried out using sodium chlorite as an oxidizing agent, the effect of color improvement is significant, and there is almost no decrease in molecular weight, unlike other chlorine-based oxidizing agents (such as NaClO). This is attributed to the fact that cell-derived impurities (proteins, lipids, and the like) in the crude product are decomposed by the reaction with sodium chlorite, whereas the PHA is hardly decomposed.

The amount of sodium chlorite used in the first decolorization may be 0.01% by weight or more, 0.02% by weight or more, 0.03% by weight or more, 0.04% by weight or more, 0.05% by weight or more, 0.06% by weight or more, 0.1% by weight or more, 0.2% by weight or more, 0.3% by weight or more, 0.4% by weight or more, or 0.5% by weight or more, and 1.3% by weight or less, 1.2% by weight or less, 1.1% by weight or less, 1% by weight or less, 0.9% by weight or less, 0.8% by weight or less, 0.7% by weight or less, or 0.6% by weight or less, based on the total weight of the crude product (solution). As a specific example, in the first decolorization, the sodium chlorite may be used in an amount of 0.01 to 1% by weight or 0.05 to 1% by weight based on the total weight of the crude product. When the amount of sodium chlorite used is within the above preferred range, the decolorization effect can be sufficiently achieved while the reduction in molecular weight is minimized.

In addition, an additional oxidizing agent may be used together with sodium chlorite (NaClO₂) as the first oxidizing agent in the first decolorization. For example, chlorine dioxide (ClO₂) may be used in an amount of 0.05 to 1% by weight based on the total weight of the crude product.

The first decolorization may be carried out under acidic pH conditions. For example, the pH range in the first decolorization may be 7 or less, 6.5 or less, 6 or less, 5.5 or less, 5 or less, 4.5 or less, or 4 or less, and 1 or more, 1.5 or more, 2 or more, 2.5 or more, 3 or more, or 3.5 or more. As a specific example, the first decolorization may be carried out at a pH of 1 to 7 or pH 2 to 7. As a more specific example, the first decolorization may be carried out at a pH of 3 to 6 or pH 3 to 5.

An acid component may be used to adjust the acidic pH. Examples of the acid component used for pH adjustment in the first decolorization include phosphoric acid, sulfuric acid, hydrochloric acid, and nitric acid, but it is not limited thereto. The crude product can be adjusted to a desired acidic pH as it comprises an appropriate amount of such an acid component.

In addition, the temperature in the first decolorization may be 40°C or higher, 45°C or higher, 50°C or higher, 55°C or higher, or 60°C or higher, and 90°C or lower, 80°C or lower, 75°C or lower, 70°C or lower, or 65°C or lower. As a specific example, the first decolorization may be carried out at a temperature of 40°C to 90°C or 50°C to 70°C.

In addition, the time required for the first decolorization may be 10 minutes or more, 30 minutes or more, or 50 minutes or more, and 3 hours or less, 2 hours or less, or 1 hour and 30 minutes or less. As a specific example, it may be 30 minutes to 1 hour and 30 minutes.

The content of impurities in the first product obtained through the first decolorization may be reduced relative to that in the previous step.

For example, the content of proteins in the first product may be 3.5% by weight or less, 3% by weight or less, or 2.7% by weight or less, and may be 0.1% by weight or more, 1% by weight or more, or 1.5% by weight or more, based on the total solids weight in the first product.

In addition, the content of lipids in the first product may be 1.5% by weight or less, 1.3% by weight or less, 1% by weight or less, or 0.7% by weight or less, and may be 0.1% by weight or more, 0.2% by weight or more, or 0.3% by weight or more, based on the total solids weight in the first product. Specifically, the first product may have a lipids content of less than 1% by weight based on the weight of the total solids content, which is significantly reduced relative to that in the previous step.

In addition, the solids content in the first product may be 1% by weight or more, 3% by weight or more, 5% by weight or more, 7% by weight or more, or 10% by weight or more, and may be 80% by weight or less, 50% by weight or less, 30% by weight or less, 20% by weight or less, or 15% by weight or less, based on the total weight of the first product.

### Second decolorization

In this step, the first product obtained by the first decolorization is subjected to second decolorization to obtain a second product.

The second decolorization may be carried out with a second oxidizing agent comprising hydrogen peroxide (H₂O₂). When hydrogen peroxide is used as an oxidizing agent in the second decolorization step once sodium chlorite has been used as an oxidizing agent in the first decolorization step, the content of impurities (proteins and lipids) after the second decolorization can be significantly reduced, and the efficiency of solubilizing impurities can be increased when the PHA solid is recovered, whereby the re-recovery ratio of impurities can also be reduced. Accordingly, the amount of enzyme used for protein decomposition can be reduced in the subsequent deproteinization step, thereby lowering the production cost.

The amount of hydrogen peroxide used in the second decolorization may be 0.01% by weight or more, 0.02% by weight or more, 0.03% by weight or more, 0.04% by weight or more, 0.05% by weight or more, 0.06% by weight or more, 0.1% by weight or more, 0.2% by weight or more, 0.3% by weight or more, 0.4% by weight or more, or 0.5% by weight or more, and 1.3% by weight or less, 1.2% by weight or less, 1.1% by weight or less, 1% by weight or less, 0.9% by weight or less, 0.8% by weight or less, 0.7% by weight or less, or 0.6% by weight or less, based on the total weight of the first product (solution). As a specific example, in the second decolorization, the hydrogen peroxide may be used in an amount of 0.01 to 1% by weight or 0.05 to 1% by weight based on the total weight of the first product. The amount of hydrogen peroxide used in the present disclosure is less than that used in the prior art, which is possible by virtue of the sequential use with sodium chlorite (first oxidizing agent).

The second decolorization may be carried out under basic pH conditions. For example, the pH range in the second decolorization may be 7 or more, 7.5 or more, 8 or more, or 8.5 or more, and may be 13 or less, 12 or less, 11 or less, 10.5 or less, 10 or less, 9.5 or less, or 9 or less. As a specific example, the second decolorization may be carried out at a pH of 7 to 13 or pH 8 to 13. As a more specific example, the second decolorization may be carried out at a pH of 8 to 11 or pH 8 to 10.

An alkaline component may be used to adjust the basic pH. Examples of the alkaline component used for pH adjustment in the second decolorization include sodium hydroxide, potassium hydroxide, lithium hydroxide, and sodium carbonate, but it is not limited thereto. The first product can be adjusted to a desired basic pH as it comprises an appropriate amount of such an alkaline component.

In addition, the temperature in the second decolorization may be 40°C or higher, 45°C or higher, 50°C or higher, 55°C or higher, or 60°C or higher, and 90°C or lower, 80°C or lower, 75°C or lower, 70°C or lower, or 65°C or lower. As a specific example, the second decolorization may be carried out at a temperature of 40°C to 90°C or 50°C to 70°C.

In addition, the time required for the second decolorization may be 10 minutes or more, 30 minutes or more, or 50 minutes or more, and 3 hours or less, 2 hours or less, or 1 hour and 30 minutes or less. As a specific example, it may be 30 minutes to 1 hour and 30 minutes.

The content of impurities in the second product obtained through the second decolorization may be reduced relative to that in the previous step.

For example, the content of proteins in the second product may be 2.5% by weight or less, 2% by weight or less, or 1.5% by weight or less, and may be 0.1% by weight or more, 0.5% by weight or more, or 1% by weight or more, based on the total solids weight in the second product.

In addition, the content of lipids in the second product may be 1% by weight or less, 0.8% by weight or less, 0.6% by weight or less, or 0.4% by weight or less, and may be 0.1% by weight or more, 0.2% by weight or more, or 0.3% by weight or more, based on the total solids weight in the second product.

In addition, the solids content in the second product may be 1% by weight or more, 3% by weight or more, 5% by weight or more, 7% by weight or more, or 10% by weight or more, and may be 80% by weight or less, 50% by weight or less, 30% by weight or less, 20% by weight or less, or 15% by weight or less, based on the total weight of the second product.

### Deproteinization

Thereafter, a step of deproteinizing the second product using a protease may be carried out.

Examples of the protease include alcalase, pepsin, trypsin, papain, chymotrypsin, aminopeptidase, and carboxypeptidase, one, two, or more of which may be used.

The amount of the protease used in the deproteinization may be 0.001% by weight or more, 0.002% by weight or more, 0.003% by weight or more, 0.004% by weight or more, 0.005% by weight or more, 0.006% by weight or more, 0.01% by weight or more, 0.015% by weight or more, 0.02% by weight or more, or 0.025% by weight or more, and may be 0.2% by weight or less, 0.15% by weight or less, 0.1% by weight or less, 0.05% by weight or less, 0.045% by weight or less, or 0.04% by weight or less, based on the total weight of the second product (solution). As an example, in the deproteinization, the protease may be used in an amount of 0.001 to 0.1% by weight or 0.005 to 0.1% by weight based on the total weight of the second product.

In addition to the protease, a lipolytic enzyme, a cell wall-decomposing enzyme, a DNA-decomposing enzyme, or the like may be further added to the second product. Examples of the lipolytic enzyme include lipase, phospholipase, cholinesterase, and phosphatase. Examples of the cell wall-decomposing enzyme include lysozyme, amylase, cellulase, maltase, saccharase, α-glycosidase, β-glycosidase, and N-glycosidase. Examples of the DNA-decomposing enzyme include ribonuclease.

The deproteinization may be carried out under basic pH conditions. For example, the pH range in the deproteinization may be 7 or more, 7.5 or more, 8 or more, or 8.5 or more, and may be 12 or less, 11 or less, 10.5 or less, 10 or less, 9.5 or less, or 9 or less.

An alkaline component may be used to adjust the basic pH. Examples of the alkaline component used for pH adjustment in the deproteinization include sodium hydroxide, potassium hydroxide, lithium hydroxide, and sodium carbonate, but it is not limited thereto. The second product can be adjusted to a desired basic pH as it comprises an appropriate amount of such an alkaline component.

In addition, the temperature in the deproteinization may be 40°C or higher, 45°C or higher, 50°C or higher, 55°C or higher, or 60°C or higher, and 90°C or lower, 80°C or lower, 75°C or lower, 70°C or lower, or 65°C or lower.

As a specific example, the deproteinization may be carried out at a pH of 7 to 11, more specifically at a pH of 8 to 10, and a temperature of 50°C to 70°C.

The content of protein may be significantly reduced through the deproteinization. For example, the content of protein may be 1% by weight or less, 0.8% by weight or less, 0.6% by weight or less, or 0.4% by weight or less, and may be 0.1% by weight or more, 0.15% by weight or more, 0.2% by weight or more, or 0.25% by weight or more, based on the total solids weight in the product upon deproteinization.

In addition, the content of lipids may be 1.5% by weight or less, 1.3% by weight or less, 1% by weight or less, or 0.7% by weight or less, and may be 0.1% by weight or more, 0.2% by weight or more, or 0.3% by weight or more, based on the total solids weight in the product upon deproteinization.

### Purified polyhydroxyalkanoate

According to the present disclosure, a purified polyhydroxyalkanoate obtained by the process described above is provided.

According to an embodiment, the purified polyhydroxyalkanoate may have a solid form and may be, for example, a solid resin. According to another embodiment, the purified polyhydroxyalkanoate may have a liquid form and may be, for example, a liquid resin or an aqueous suspension.

The polyhydroxyalkanoate prepared according to the present disclosure can have improved color. According to an embodiment, the polyhydroxyalkanoate may have a b* value of less than 15 in the CIELAB color coordinates. For example, the b* value of the purified polyhydroxyalkanoate in the CIELAB color coordinates may be 14 or less, 13.5 or less, 13 or less, and may be 0 or more, 1 or more, 5 or more, or 10 or more.

In addition, the polyhydroxyalkanoate may have a glass transition temperature (Tg) of, for example, -45°C to -10°C, -35°C to -10°C, -35°C to -15°C, -35°C to -20°C, or -30°C to -20°C.

In addition, the melting temperature (Tm) of the polyhydroxyalkanoate, for example, may not be measured or, for example, may be 100°C to 170°C, 100°C to 160°C, 110°C to 160°C, or 120°C to 150°C.

The polyhydroxyalkanoate of the present disclosure may comprise an amorphous polyhydroxyalkanoate, a semi-crystalline polyhydroxyalkanoate, and a mixture thereof. For example, the crystallization temperature (Tc) of the polyhydroxyalkanoate may not be measured, or the crystallization temperature (Tc) may be measured to be 60°C to 120°C, specifically, 60°C to 110°C, 70°C to 120°C, or 75°C to 115°C.

The repeat units that constitute the polyhydroxyalkanoate may comprise at least one selected from the group consisting of 2-hydroxybutyrate (2-HB), 3-hydroxybutyrate (3-HB), 3-hydroxypropionate (3-HP), 3-hydroxyvalerate (3-HV), 3-hydroxyhexanoate (3-HH), 3-hydroxyheptanoate (3-HHep), 3-hydroxyoctanoate (3-HO), 3-hydroxynonanoate (3-HN), 3-hydroxydecanoate (3-HD), 3-hydroxydodecanoate (3-HDd), 4-hydroxybutyrate (4-HB), 4-hydroxyvalerate (4-HV), 5-hydroxyvalerate (5-HV), and 6-hydroxyhexanoate (6-HH).

According to an embodiment, the polyhydroxyalkanoate may comprise a 4-hydroxybutyrate (4-HB) repeat unit. Specifically, it may comprise a copolymer comprising a 4-HB repeat unit. For example, the content of a 4-hydroxybutyrate (4-HB) repeat unit in the polyhydroxyalkanoate may be 0.1% by weight or more, 1% by weight or more, 5% by weight or more, 10% by weight or more, 20% by weight or more, 30% by weight or more, 40% by weight or more, 50% by weight or more, or 60% by weight or more, and 100% by weight or less, 99% by weight or less, 90% by weight or less, 80% by weight or less, 70% by weight or less, or 60% by weight or less, based on the total weight of the polyhydroxyalkanoate. As a specific example, the polyhydroxyalkanoate may comprise a 4-hydroxybutyrate (4-HB) repeat unit in an amount of 25% by weight or more.

In addition, the polyhydroxyalkanoate may have a weight average molecular weight (Mw) of, for example, 50,000 or more, 100,000 or more, 200,000 or more, 300,000 or more, 400,000 or more, or 500,000 or more, and may be 1,200,000 or less, 900,000 or less, 800,000 or less, 700,000 or less, or 600,000 or less.

In addition, the polyhydroxyalkanoate may have a purity of 95% or more, 97% or more, 98% or more, 98.5% or more, 98.55% or more, 98.6% or more, or 99.0% or more.

### Mode for the Invention

Hereinafter, the present disclosure will be described in more detail through the following examples. However, these examples are provided only for illustration purposes, and the present disclosure is not limited thereto.

### Preparation Example 1: Preparation of PHA fermentation broth

A genetically engineered *E*. *coli* strain was used to prepare PHA fermentation broth. First, the PHA-producing strain was seed cultured to secure a certain amount, and main fermentation was then carried out. For the fermentation of the PHA-producing strain, glucose or raw sugar was used as a carbon source, and various mineral components were added as nutrients. A sodium hydroxide solution or ammonia gas was used to adjust pH during the fermentaiton. The fermentation process using the PHA-producing strain was carried out at 30 to 40°C and a pH of 6 to 9.

### Example 1: Preparation of a purified polyhydroxyalkanoate

### Step (1): Solid-liquid separation and cell crushing

The PHA fermentation broth was subjected to a solid-liquid separation using a mechanical separator to separate the culture medium impurities and biomass containing the PHA. The separated biomass was adjusted to have a total solids content of 10 to 15% by weight. A surfactant (sodium laureth sulfate, 30%, Miwon Corporation) was added in an amount of 0.13% by weight based on the total weight of the reaction solution. Upon sufficient stirring, physical cell crushing was carried out using a high-pressure homogenizer (Bertoli) (400 to 800 bar, 1 pass). The PHA solution thus obtained was subjected to a second solid-liquid separation to separate the PHA-containing solids and cell-derived impurities.

### Step (2): Decolorization

An acid (10% H₃PO₄, Deoksan Scientific) was added to the separated PHA solution to obtain a reaction solution with a pH adjusted to approximately 3.5 to 4. Sodium chlorite (NaClO₂) was added in an amount of 0.55% by weight (approximately 5% by weight based on the solids content) based on the total weight of the reaction solution, and then first decolorization was carried out at 60°C for 1 hour.

An alkali (40% NaOH, Deoksan Scientific) was added to the first decolorized slurry to obtain a reaction solution with a pH adjusted to approximately 9 to 9.5. Hydrogen peroxide (H₂O₂) was added in an amount of 0.22% by weight (approximately 2% by weight based on the solids content) based on the total weight of the reaction solution, and then second decolorization was carried out at 60°C for 1 hour.

### Step (3): Deproteinization

An alkaline protease (alcalase, Novozyme) was added to the reaction solution obtained in the decolorization step in an amount of 0.044% by weight (approximately 0.4% by weight based on the solids content) based on the total weight of the reaction solution, and deproteinization was carried out at a pH of about 9.5 for 2 hours. After the deproteinization, the solid (PHA) recovered was washed with water and then dried to obtain a final purified polyhydroxyalkanoate resin.

### Example 2

The same procedure as in Example 1 was repeated to obtain a purified polyhydroxyalkanoate resin, except that the amounts of the first oxidizing agent (NaClO₂) and the second oxidizing agent (H₂O₂) were each changed as shown in Table 1 below.

### Comparative Example 1

The same procedure as in Example 1 was repeated to obtain a purified polyhydroxyalkanoate resin, except that decolorization was carried out under the conditions shown in Table 1 below using only hydrogen peroxide (H₂O₂) as an oxidizing agent.

### Comparative Example 2

The same procedure as in Example 1 was repeated to obtain a purified polyhydroxyalkanoate resin, except that decolorization was carried out under the conditions shown in Table 1 below using only sodium chlorite (NaClO₂) as an oxidizing agent.

### Comparative Example 3

The same procedure as in Example 1 was repeated to obtain a purified polyhydroxyalkanoate resin, except that decolorization was carried out under the conditions shown in Table 1 below using only sodium hypochlorite (NaClO) as an oxidizing agent.

### Comparative Example 4

The same procedure as in Example 1 was repeated to obtain a purified polyhydroxyalkanoate resin, except that decolorization was carried out under the conditions shown in Table 1 below using sodium hypochlorite (NaClO) as the first oxidizing agent and hydrogen peroxide (H₂O₂) as the second oxidizing agent.

The oxidizing agents and the reaction conditions used in the Examples and Comparative Examples are summarized in the table below.

**[Table 1]**

| | Type of oxidizing agent | | Decolorization reaction conditions | | Amount of oxidizing agent* | |
|---|---|---|---|---|---|---|
| C. Ex. 1 | H₂O₂ | | pH 9-9.5, 60°C, 2 hrs | | 0.77 (7) | |
| C. Ex. 2 | NaClO₂ | | pH 3.5-4, 60°C, 2 hrs | | 0.55 (5) | |
| Ex. 1 | NaClO₂ | H₂O₂ | pH 3.5-4, 60°C, 1 hr | pH 9-9.5, 60°C, 1 hr | 0.55 (5) | 0.22 (2) |
| Ex. 2 | NaClO₂ | H₂O₂ | pH 3.5-4, 60°C, 1 hr | pH 9-9.5, 60°C, 1 hr | 0.22 (2) | 0.33 (3) |
| C. Ex. 3 | NaClO | | pH 9-9.5, 25°C, 0.5 hr | | 1.1 (10) | |
| C. Ex. 4 | NaClO | H₂O₂ | pH 9-9.5, 25°C, 0.5 hr | pH 9-9.5, 25°C, 1.5 hrs | 1.1 (10) | 0.55 (5) |
| * Amount of oxidizing agent (unit: % by weight): The amount of oxidizing agent added based on the total weight of the reaction solution (the amount of oxidizing agent added based on the solids content is shown in parentheses) | | | | | | |

### Test Example 1: Color and molecular weight

The polyhydroxyalkanoate resins obtained upon deproteinization in the Examples and Comparative Examples were each tested as follows.

### (1) Color (b* value)

The b* value of the PHA solid was measured using a spectrophotometer (Konica Minolta). About 8 to 10 g of the PHA solid was placed on a petri dish for the spectrophotometer, and the color was measured.

### (2) Molecular weight

The molecular weight of the PHA was measured by gel permeation chromatography (GPC). Each PHA was dissolved by adding chloroform equivalent to 50 times the PHA solid content. Impurities were removed using a 0.45-µm syringe filter, and GPC analysis was carried out to calculate the weight average molecular weight (Mw).

The results are shown in the table below.

**[Table 2]**

| | b* value | Mw | Summarized result |
|---|---|---|---|
| C. Ex. 1 | 16.2 | 541,408 | b* of 15 or higher |
| C. Ex. 2 | 15.6 | 560,771 | b* of 15 or higher |
| Ex. 1 | 10.0 | 535,546 | Color improved without a decrease in molecular weight |
| Ex. 2 | 12.4 | 563,062 | Color improved without a decrease in molecular weight |
| C. Ex. 3 | 7.4 | 384,926 | Molecular weight decreased |
| C. Ex. 4 | 7.4 | 474,711 | Molecular weight decreased |

As can be seen from the above table, when only one of hydrogen peroxide (H₂O₂) and sodium chlorite (NaClO₂) was used as in Comparative Examples 1 and 2, the b* value of the final PHA resin was measured to be 15 or higher, indicating that there was no effect of color improvement.

In contrast, when sodium chlorite (NaClO₂) and hydrogen peroxide (H₂O₂) were sequentially used as in Examples 1 and 2, the b* value of the final PHA resin was less than 15, resulting in a significant improvement in color. In particular, the molecular weight (Mw) of the PHA resin obtained in Example 1 showed little difference from those of Comparative Examples 1 and 2, indicating that the color was improved without a decrease in molecular weight.

In contrast, when only sodium hypochlorite (NaClO) was used as an oxidizing agent as in Comparative Example 3, the effect of color improvement was confirmed, while the molecular weight was significantly decreased (approximately 29% lower than Comparative Example 1). Even when it is used sequentially with hydrogen peroxide as in Comparative Example 4, there was an effect of color improvement, while the molecular weight was significantly decreased.

### Test Example 2: Analysis of impurities content

The content of cell-derived impurities (proteins and lipids) in the product prepared in each step of the Examples and Comparative Examples was analyzed. Specifically, the PHA slurry solution obtained in each purification step was centrifuged to separate the solid and liquid components. Then, protein analysis was carried out using the Dumas analysis method, and lipid extraction was carried out using diethyl ether. The results are shown in Tables 3 and 4 below.

**[Table 3]**

| Comparative Example 1 | | Solid component (g) | | | Liquid component (g) | | Total (g) | |
|---|---|---|---|---|---|---|---|---|
| | | Total | Proteins | Lipids | Total | Proteins | Total | Proteins |
| 1 | Upon solid-liquid separation | 98.9 | 1.73 (1.75%) | 0.41 (0.42%) | 1.1 | 0.59 | 100.0 | 2.32 |
| 2 | Upon decolorization (H₂O₂) | 96.4 | 1.33 (1.38%) | 0.97 (1.01%) | 1.9 | 1.04 | 98.3 | 2.36 |
| 3 | Upon deproteinization | 90.2 | 0.23 (0.25%) | 0.49 (0.54%) | 4.3 | 2.18 | 94.6 | 2.40 |
| 4 | Final PHA resin | 88.3 | 0.58 (0.66%) | 0.67 (0.76%) | | | | |

**[Table 4]**

| Example 2 | | Solid component (g) | | | Liquid component (g) | | Total (g) | |
|---|---|---|---|---|---|---|---|---|
| | | Total | Proteins | Lipids | Total | Proteins | Total | Proteins |
| 1 | Upon solid-liquid separation | 99.1 | 2.26 (2.28%) | 0.56 (0.57%) | 0.9 | 0.48 | 100.0 | 2.74 |
| 2-1 | Upon first decolorization (NaClO₂) | 99.5 | 2.61 (2.63%) | 0.51 (0.51%) | 3.0 | 0.17 | 102.5 | 2.79 |
| 2-2 | Upon second decolorization (H₂O₂) | 97.4 | 1.34 (1.38%) | 0.31 (0.32%) | 4.7 | 0.89 | 102.0 | 2.23 |
| 3 | Upon deproteinization | 95.4 | 0.27 (0.28%) | 0.46 (0.48%) | 6.1 | 1.93 | 101.5 | 2.19 |
| 3 | Final PHA resin | 89.7 | 0.25 (0.31%) | 0.49 (0.55%) | | | | |

As can be seen from Table 3 above, in Comparative Example 1 in which hydrogen peroxide was used as an oxidizing agent, proteins were decomposed, while lipids were hardly decomposed. In addition, it is understood that the increase in the b* value of the final PHA resin was affected by the increase in the content of impurities (proteins and lipids) during the recovery of the solid after purification.

In contrast, as can be seen from Table 4 above, in Example 2 in which a first oxidizing agent (NaClO₂) and a second oxidizing agent (H₂O₂) were sequentially used, upon the second decolorization, the contents of proteins and lipids were each significantly reduced. Further, the rate at which impurities were re-recovered during the recovery of the solid was lower than that in Comparative Example 1. It is understood that the decolorization by the first oxidizing agent (NaClO₂) caused structural breakdown and decomposition of proteins and lipids, and the decolorization by the second oxidizing agent (H₂O₂) caused a rapid increase in the decomposition rates of proteins and lipids, resulting in rapid decomposition of cell-derived impurities. As a result, the content of impurities remaining in the final PHA resin was lowered, thereby leading to a decrease in the b* value.

### Test Example 3: Analysis of the purified PHA

The polyhydroxyalkanoate (PHA) resins finally obtained in the Examples and Comparative Examples were each tested as follows.

### (1) b* value

The b* value of the PHA solid was measured using a spectrophotometer (Konica Minolta). About 8 g of the PHA solid was placed on a petri dish for the spectrophotometer, and the color was measured.

### (2) 4-HB content

The content of 4-HB in the PHA was measured by gas chromatography (GC) after a butanolysis reaction. γ-Butyrolactone (GBL) was used as a standard reagent for the analysis of 4-HB content. The same butanolysis reaction was carried out for conversion to butyl ester, and it was quantified using concentration-specific standards. A butanolysis solution (n-butanol and 4 M HCl in dioxane) and an internal standard solution (diphenylmethane) were added to 20 mg of a PHA sample, which was sufficiently dissolved by sonication at 70°C for 1 hour. The dissolved sample was reacted in a hot water bath at 95°C for 6 hours. Water was added to induce layer separation, and the upper layer (organic layer) was separated and analyzed by GC to calculate the 4-HB content.

### (3) Purity of the PHA

The content of impurities in the dried PHA solid component was measured, and the PHA purity was calculated using the following equation. The contents of crude proteins, crude lipids, and other impurities (ash) were measured as impurities. The content of impurities was measured on a total dry weight basis. PHA purity (%) = 100 - (crude proteins (%) + crude lipids (%) + other impurities (ash) (%))

The results are shown in the table below.

**[Table 5]**

| | Final PHA resin | |
|---|---|---|
| | Comparative Example 1 | Example 2 |
| b* value | About 18 (range of 16 to 20) | About 13 (range of 12 to 14) |
| 4-HB content | > 40% by weight | > 40% by weight |
| PHA purity | 98.54% | 99.14% |

As can be seen from the above table, the final PHA resin obtained in Example 2 according to the present disclosure was an amorphous resin having a 4-HB content exceeding 40% by weight, a high PHA purity of 98.5% or more, and a b* value, which is an indicator of a yellow index, of less than 15, which was significantly lower than that of Comparative Example 1, indicating that the color was excellent.

## Claims

1. A process for preparing a purified polyhydroxyalkanoate, which comprises:
preparing a crude product comprising a polyhydroxyalkanoate (PHA);
first decolorizing the crude product to obtain a first product; and
second decolorizing the first product to obtain a second product,
wherein the first decolorization is carried out with a first oxidizing agent comprising sodium chlorite (NaClO₂).

2. The process for preparing a purified polyhydroxyalkanoate of claim 1, wherein the second decolorization is carried out with a second oxidizing agent comprising hydrogen peroxide (H₂O₂).

3. The process for preparing a purified polyhydroxyalkanoate of claim 1, wherein the first decolorization is carried out at a pH of 2 to 7, and the second decolorization is carried out at a pH of 8 to 13.

4. The process for preparing a purified polyhydroxyalkanoate of claim 1, wherein, in the first decolorization, the sodium chlorite is used in an amount of 0.01 to 1% by weight based on the total weight of the crude product.

5. The process for preparing a purified polyhydroxyalkanoate of claim 2, wherein, in the second decolorization, the hydrogen peroxide is used in an amount of 0.01 to 1% by weight based on the total weight of the first product.

6. The process for preparing a purified polyhydroxyalkanoate of claim 1, wherein the first product has a content of lipids of less than 1% by weight based on the weight of the total solids content.

7. The process for preparing a purified polyhydroxyalkanoate of claim 1, which further comprises deproteinizing the second product using a protease.

8. The process for preparing a purified polyhydroxyalkanoate of claim 7, wherein, in the deproteinization, the protease is used in an amount of 0.001 to 0.1% by weight based on the total weight of the second product.

9. The process for preparing a purified polyhydroxyalkanoate of claim 7, wherein the deproteinization is carried out at a pH of 7 to 11.

10. The process for preparing a purified polyhydroxyalkanoate of claim 1, wherein the crude product comprising a polyhydroxyalkanoate is prepared by a method comprising:
performing a solid-liquid separation of a fermentation broth comprising a polyhydroxyalkanoate (PHA) to obtain biomass;
mixing an additive comprising a surfactant with the biomass to obtain a suspension;
crushing the biomass in the suspension to obtain a slurry; and
performing a solid-liquid separation of the slurry.

11. A polyhydroxyalkanoate, which is prepared by the process of claim 1 and has a b* value of less than 15 in the CIELAB color coordinates.

12. The polyhydroxyalkanoate of claim 11, wherein the crystallization temperature (Tc) of the polyhydroxyalkanoate is not measured or is measured to be 60°C to 120°C.

13. The polyhydroxyalkanoate of claim 11, wherein the repeat units that constitute the polyhydroxyalkanoate comprise at least one selected from the group consisting of 2-hydroxybutyrate (2-HB), 3-hydroxybutyrate (3-HB), 3-hydroxypropionate (3-HP), 3-hydroxyvalerate (3-HV), 3-hydroxyhexanoate (3-HH), 3-hydroxyheptanoate (3-HHep), 3-hydroxyoctanoate (3-HO), 3-hydroxynonanoate (3-HN), 3-hydroxydecanoate (3-HD), 3-hydroxydodecanoate (3-HDd), 4-hydroxybutyrate (4-HB), 4-hydroxyvalerate (4-HV), 5-hydroxyvalerate (5-HV), and 6-hydroxyhexanoate (6-HH).
